# EUROPÄISCHE PATENTANMELDUNG

(11) **EP 0 921 112 A1**
(43) Veröffentlichungstag der Anmeldung: **09.06.1999**
(21) Anmeldenummer: 98122571.7
(22) Anmeldetag: 03.12.1998
(51) Int. Cl.: C07C 51/42, C07C 57/04

(54) **Verfahren zur Aufkonzentrierung von Methacrylsäure in wasserhaltigen Lösungen**

(30) Priorität: 05.12.1997 DE 19754118
(71) Anmelder: BASF AKTIENGESELLSCHAFT, 67056 Ludwigshafen (DE)
(72) Erfinder: Ulbrich, Michael-Dieter, Dr., 67251 Freinsheim (DE); Meckl, Stefan, Dr., 67157 Wachenheim (DE); Sauer, Friedrich, 67271 Obersülzen (DE); Schraut, Armin, Dr., 64625 Bensheim (DE); Martin, Friedrich-Georg, Dr., 69124 Heidelberg (DE)
(74) Vertreter: Isenbruck, Günter, Dr.

(57) **Zusammenfassung**

In einem Verfahren zur Aufkonzentrierung von Methacrylsäure in wasserhaltigen Lösungen kühlt man die Lösung ab, bis sich zwei Phasen ausbilden, und trennt die an Methacrylsäure reichere Lösung von der an Methacrylsäure ärmeren Lösung ab. Vorzugsweise wird die Lösung auf 14 bis -2°C abgekühlt und eine wasserhaltige Methacrylsäurelösung mit 13 bis 70 Gew.-% Methacrylsäure verwendet.

## Beschreibung

Die vorliegende Erfindung betrifft ein Verfahren zur Aufkonzentrierung von Methacrylsäure in wasserhaltigen Methacrylsäurelösungen.

Die Aufkonzentrierung von Methacrylsäure in wasserhaltigen Lösungen, insbesondere auf Lösungen mit mehr als 20 Gew.-% Methacrylsäure, ist aufwendig, da das System Methacrylsäure/Wasser bei etwa 20 Gew.-% Methacrylsäure ein Azeotrop bildet (vgl. Gmehling/Onken, Chemistry Data Series, Afanasenkova et al., J. Appl. Chem. USSR, 46 (1973)). Mit einer einfachen Destillation kann somit aus einer verdünnten, wäßrigen Methacrylsäurelösung eine Lösung mit maximal 20 Gew.-% Methacrylsäure erzeugt werden. Andererseits verursacht bei einer destillativen Aufkonzentrierung von Methacrylsäurelösungen auf Lösungen mit mehr als 20 Gew.-% Methacrylsäure die thermische Belastung, z.B. bei Einsatz einer Kolonne mit trennwirksamen Einbauten, oft Polymerisationsprobleme.

Da die destillative Überschreitung des Azeotrops mit einer Zweidruckdestillation bzw. Extraktivdestillation aufwendig ist, werden üblicherweise Flüssig/Flüssig-Extraktionen mit destillativer Aufarbeitung des Extraktes verwendet (vgl. Ullmanns Encyclopedia, Band A 16, S. 446).

Die Aufgabe der vorliegenden Erfindung bestand vor diesem Hintergrund darin, ein Verfahren zu schaffen, bei dem auf einfachem Weg eine Aufkonzentrierung von verdünnten Methacrylsäurelösungen erreicht wird.

Überraschenderweise wurde gefunden, daß das Phasengleichgewicht von Methacrylsäure und Wasser eine Mischungslücke, d.h. zwei flüssige Phasen, über einen weiten Konzentrationsbereich zeigt, und sich hierbei eine an Methacrylsäure reichere Lösung und eine an Methacrylsäure ärmere Lösung ausbildet.

Das Fest-Flüssig-Phasengleichgewicht zwischen Methacrylsäure und Wasser ist bei D.A. Chubarov, S.M. Danov und G.V. Brovkina in Zhurnal Prikladnoi Khimii, Band 51/8, S. 1899-1900, August 1978, beschrieben. Dabei wird auf eine vermutete Mischungslücke hingewiesen, wobei jedoch keine Messungen hierzu durchgeführt wurden.

Somit betrifft die Erfindung ein Verfahren zur Aufkonzentrierung von Methacrylsäure in wasserhaltigen Methacrylsäurelösungen, das dadurch gekennzeichnet ist, daß man die Lösung abkühlt, bis sich zwei Phasen ausbilden, und die an Methacrylsäure reichere Lösung von der an Methacrylsäure ärmeren Lösung abtrennt. Bevorzugte Ausführungsformen der Erfindung sind in der nachfolgenden Beschreibung, den Unteransprüchen, den Figuren und den Beispielen beschrieben.

Die Figuren zeigen:
- Fig. 1: grafisch die experimentell ermittelten Meßwerte bei der Bestimmung des Fest-Flüssig- und Flüssig-Flüssig-Phasengleichgewichts zwischen Methacrylsäure und Wasser;
- Fig. 2: eine bevorzugte Ausführungsform der vorliegenden Erfindung;
- Fig. 3: eine bevorzugte Ausführungsform der Erfindung mit einem eine Mischungslücke schließenden Zusatz;
- Fig. 4: eine bevorzugte Ausführungsform der Erfindung mit oder ohne einem eine Mischungslücke schließenden Zusatz sowie mit Rückführung der an Methacrylsäure ärmeren Lösung; und
- Fig. 5: eine bevorzugte Ausführungsform der Erfindung mit Vorkonzentrierung der wasserhaltigen Methacrylsäurelösung.

Geeignete Lösungen, in denen erfindungsgemäß die Methacrylsäure aufkonzentriert werden kann, enthalten vorzugsweise 13 bis 70 Gew.-%, insbesondere 15 bis 68 Gew.-%, stärker bevorzugt 15 bis 40 Gew.-%, am meisten bevorzugt 15 bis 20 Gew.-% Methacrylsäure und 30 bis 87 Gew.-%, insbesondere 32 bis 85 Gew.-%, stärker bevorzugt 60 bis 85 Gew.-%, am meisten bevorzugt 80 bis 85 Gew.-% Wasser, jeweils bezogen auf 100 Gew.-% Methacrylsäure und Wasser. Die Methacrylsäure kann auch in wasserhaltigen Lösungen aufkonzentriert werden, die neben der Methacrylsäure und dem Wasser weitere Verbindungen oder Nebenkomponenten enthalten, z.B. Verunreinigungen, die aus dem Herstellungsprozeß der Methacrylsäure stammen. Eine besonders geeignete Lösung liegt vor, wenn man ein Reaktionsgasgemisch, wie es bei der katalytischen Gasphasenoxidation von C₄-Alkanen, -Alkenen, -Alkanalen und/oder -Alkanolen und/oder Vorstufen davon, z.B. Isobuten, Isobutan, Methacrolein, Isobutyraldehyd, Isobuttersäure oder Methyl-tert.-butylether (MTBE) entsteht, in eine Lösung überführt, z.B. durch Kondensation oder Absorption in Wasser. Liegen Nebenkomponenten in der aufzukonzentrierenden Methacrylsäurelösung vor, dann beträgt deren Menge vorzugsweise nicht mehr als 10 Gewichtsteile, insbesondere liegt sie zwischen 0 und 10 Gewichtsteilen, vorzugsweise bei 6 bis 9 Gewichtsteilen, jeweils bezogen auf 100 Gewichtsteile Methacrylsäure und Wasser. Verhindern diese Verunreinigungen oder Nebenkomponenten die Bildung von zwei Phasen, so kann durch Zugabe geeigneter Verbindungen die Bildung von zwei flüssigen Phasen erreicht werden. Geeignete Zusätze sind alle mit Wasser eine Mischungslücke bildenden Komponenten, vorzugsweise aliphatische und/oder aromatische Kohlenwasserstoffe, insbesondere C₄-C₁₄-Kohlenwasserstoffe, die auch substituiert sein können und/oder Heteroatome aufweisen können. Insbesondere sind Stoffe, die durch Gasphasenoxidation zu Methacrylsäure umgesetzt werden, wie 1-Buten, Isobuten, Isobutyraldehyd, Isobuttersäure, MTBE und/oder Methacrolein, geeignet. Besonders vorteilhaft ist die Verwendung von Estern der Methacrylsäure, insbesondere Methylmethacrylat, da diese bei einer nachfolgenden Veresterung von Methacrylsäure keinen Fremdstoff im Verfahren darstellen. Die geeignete Menge an die Mischungslücke bildenden Zusätzen hängt von der Menge an Verunreinigungen oder Nebenkomponenten ab und kann im Rahmen fachüblicher Versuche leicht vom Fachmann ermittelt werden. Üblicherweise beträgt sie nicht mehr als 30 Gewichtsteile, insbesondere 1 bis 20 Gewichtsteile, bevorzugt 1 bis 10 Gewichtsteile, jeweils bezogen auf 100 Gewichtsteile Wasser, Methacrylsäure und Verunreinigungen/Nebenkomponenten.

Erfindungsgemäß wird die aufzukonzentrierende Lösung solange abgekühlt, bis sich zwei Phasen ausbilden. Zweckmäßigerweise wird auf 14 bis -2 °C, insbesondere auf 10 bis 0 °C, am meisten bevorzugt auf 8 bis 3 °C abgekühlt. Bei Anwesenheit von Verunreinigungen hängt die Temperatur, auf die abgekühlt wird, von deren Gehalt ab.

Die Trennung der zwei, sich bildenden Phasen unterliegt keiner besonderen Beschränkung und kann nach allen Verfahren erfolgen, die sich zur Trennung von zwei flüssigen Phasen eignen. Zweckmäßigerweise wird die Trennung mittels Settler- oder Schwerkraftabscheider durchgeführt.

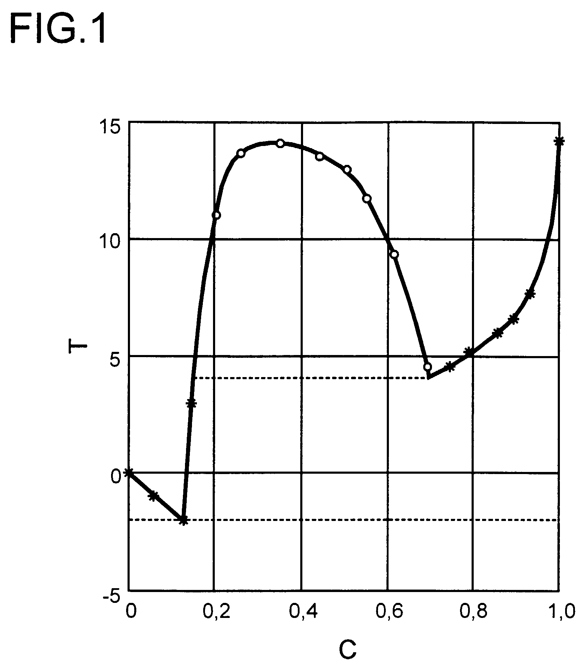

Durch einfaches Abkühlen einer wasserhaltigen Methacrylsäurelösung wird somit eine konzentrierte, an Methacrylsäure reichere Lösung und eine verdünnte, an Methacrylsäure ärmere Lösung gewonnen. In Fig. 1 sind grafisch die experimentell ermittelten Meßwerte bei der Bestimmung des Fest-Flüssig- und Flüssig-Flüssig-Phasengleichgewichts zwischen Methacrylsäure und Wasser in Abhängigkeit von der Temperatur und dem Anteil von Methacrylsäure zu Wasser gezeigt. Hierbei bezeichnet T die Temperatur in °C und C den Anteil von Methacrylsäure zu Wasser in Gew.-% Methacrylsäure bezogen auf 100 Gew.-% Methacrylsäure und Wasser. Gemäß Fig. 1 beträgt die größte Breite der Mischungslücke 15 bis 68 Gew.-% Methacrylsäure und 32 bis 85 Gew.-% Wasser, während die kritische Mischungstemperatur bei etwa 14,1 °C bei etwa 35 Gew.-% Methacrylsäure und etwa 65 Gew.-% Wasser liegt. Im Bereich der Mischungslücke liegt die Konzentration an Methacrylsäure in der an Methacrylsäure reicheren Lösung zwischen 35 und 68 Gew.-% Methacrylsäure und in der an Methacrylsäure ärmeren Lösung zwischen 15 und 35 Gew.-% Methacrylsäure, jeweils bezogen auf 100 Gew.-% Methacrylsäure und Wasser.

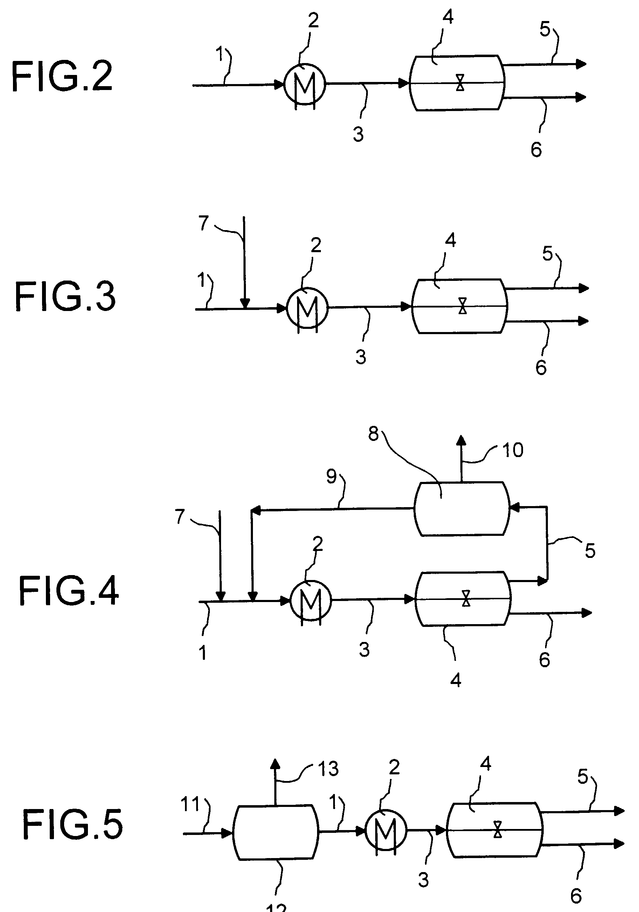

Fig. 2 zeigt eine bevorzugte Ausgestaltung der Erfindung, bei der eine aufzukonzentrierende, einphasige, wasserhaltige Methacrylsäurelösung 1 in der mit Bezugsziffer 2 bezeichneten Stufe abgekühlt wird, bis sich zwei Phasen ausbilden. Die zweiphasige Lösung 3 wird dann in einer geeigneten Vorrichtung 4, z.B. einem Settler, in die beiden Phasen getrennt. Über Leitung 5 wird die an Methacrylsäure ärmere Lösung abgeführt, während über Leitung 6 die an Methacrylsäure reichere Lösung abgeführt wird. In den Fig. 3 bis 5 bezeichnen gleiche Bezugsziffern wie in Fig. 2 das Gleiche, sofern nichts anderes angegeben ist. In Fig. 3 wird einer einphasigen Methacrylsäurelösung 1, die die Mischungslücke schließende Nebenkomponenten enthält, vor dem Abkühlen 2 ein die Mischungslücke öffnender Zusatz, z.B. Methylmethacrylat, über Leitung 7 zugeführt und wie in Fig. 1 dargestellt weiter verfahren. In Fig. 4 wird wie in Fig. 3 verfahren und nach der Abtrennung der beiden flüssigen Phasen in der Vorrichtung 4 die an Methacrylsäure ärmere Lösung aus Leitung 5 mittels eines einfachen thermischen Trennverfahrens 8, z.B. einer Rektifikation oder Destillation, auf die azeotrope Zusammensetzung aufkonzentriert und diese über Leitung 9 erneut dem Abkühlungsschritt zugeführt. In Fig. 5 wird die aufzukonzentrierende, einphasige Lösung 1 aus einer verdünnteren Methacrylsäurelösung 11 durch Aufkonzentrierung 12, z.B. Destillation, bis zur azeotropen Zusammensetzung gewonnen und anschließend wie in Fig. 2 verfahren. Über Leitung 13 wird das bei der azeotropen Aufkonzentrierung anfallende Wasser abgeführt. Die in den Fig. 2 und 3 gezeigten Verfahren sind in den Beispielen 1 bis 6 näher beschrieben.

Gegenüber den bekannten Verfahren bietet das erfindungsgemäße Verfahren den Vorteil, daß kein Fremdstoff, z.B. kein Extraktionsmittel, erforderlich ist. Daneben wird die Methacrylsäurelösung bei der Aufkonzentrierung keinen hohen Temperaturen ausgesetzt, wodurch eine mögliche Polymerisation verhindert wird. Des weiteren ist der apparative Aufwand für die Abkühlung und Trennung gering.

Die Erfindung wird anhand der folgenden Beispiele, die bevorzugte Ausführungsformen der Erfindung darstellen, näher erläutert.

### Beispiel 1

### (Aufkonzentrierung von unter-azeotroper Methacrylsäure(MAS)-Lösung - Fig. 2)

Eine wäßrige Lösung mit 19 Gew.-% MAS (Gesamtmasse 980 g) wurde in einem 1 l Glasbehälter mit Wendelrührer einphasig flüssig vorgelegt und abgekühlt. Bei einer Temperatur von 5 °C wurde am Boden des Glasbehälters 74 g einer klaren zweiten flüssigen Phase abgezogen. Diese zweite flüssige Phase enthielt 68,4 Gew.-% MAS, während in der verbleibenden flüssigen Phase 14,5 Gew.-% MAS gemessen wurden.

### Beispiel 2

### (Aufkonzentrierung von über-azeotroper MAS-Lösung - Fig. 2)

Eine wäßrige Lösung mit 40 Gew.-% MAS (Gesamtmasse 950 g) wurde in einem 1 l Glasbehälter mit Wendelrührer einphasig flüssig vorgelegt und abgekühlt. Bei einer Temperatur von 10 °C wurde am Boden des Glasbehälters 492 g einer klaren zweiten flüssigen Phase abgezogen. Diese zweite flüssige Phase enthielt 59 Gew.-% MAS, während in der verbleibenden flüssigen Phase 19 Gew.-% MAS gemessen wurden.

### Beispiel 3

### (Aufkonzentrierung von MAS/ES-Lösung durch Zugabe von MMA - Fig. 3)

203,5 g wäßrige Lösung mit 3,1 Gew.-% Essigsäure (ES) und 30,9 Gew.-% MAS wurde in einem Glasgefäß auf 10 °C abgekühlt. Nach Zugabe von 8,5 g Methylmethacrylat (MMA) konnte am Boden des Gefäßes 73,8 g einer zweiten klaren flüssigen Phase abgezogen werden. Diese zweite flüssige Phase enthielt 2,95 Gew.-% ES, 64,76 Gew.-% MAS und 9,81 Gew.-% MMA. Die verbleibende flüssige Phase enthielt 3,2 Gew.-% ES, 13,6 Gew.-% MAS und 0,56 Gew.-% MMA.

### Beispiel 4

### (Aufkonzentrierung von MAS/ES-Lösung durch Zugabe von MMA - Fig. 3)

201,9 g wäßrige Lösung mit 4 Gew.-% Essigsäure (ES) und 40 Gew.-% MAS wurde in einem Glasgefäß auf 10 °C abgekühlt. Nach Zugabe von 11,6 g MMA konnte am Boden des Gefäßes 97,6 g einer zweiten klaren flüssigen Phase abgezogen werden. Diese zweite flüssige Phase enthielt 3,8 Gew.-% ES, 61,94 Gew.-% MAS und 9,44 Gew.-MMA. Die verbleibende flüssige Phase enthielt 4,1 Gew.-% ES, 15 Gew.-% MAS und 0,67 Gew.-% MMA.

### Beispiel 5

### (Aufkonzentrierung von MAS/ES-Lösung durch Zugabe von MMA - Fig. 3)

203,8 g wäßrige Lösung mit 3 Gew.-% Essigsäure (ES) und 29,9 Gew.-% MAS wurde in einem Glasgefäß auf 5 °C abgekühlt. Nach Zugabe von 6,5 g MMA konnte am Boden des Glasgefäßes 61,3 g einer zweiten klaren flüssigen Phase abgezogen werden. Diese zweite flüssige Phase enthielt 2,8 Gew.-% ES, 68,9 Gew.-% MAS und 8,8 Gew.-% MMA. Die verbleibende flüssige Phase enthielt 3,1 Gew.-% ES, 13,87 Gew.-% MAS und 0,53 Gew.-% MMA.

### Beispiel 6

### (Aufkonzentrierung von MAS/ES-Lösung durch Zugabe von MMA - Fig. 3)

223,9 g wäßrige Lösung mit 4 Gew.-% Essigsäure (ES) und 39,9 Gew.-% MAS wurde in einem Glasgefäß auf 5 °C abgekühlt. Nach Zugabe von 9,5 g MMA konnte am Boden des Gefäßes 115,8 g einer zweiten klaren flüssigen Phase abgezogen werden. Diese zweite flüssige Phase enthielt 4,1 Gew.-% ES, 65,1 Gew.-% MAS und 7,7 Gew.-% MMA. Die verbleibende flüssige Phase enthielt 4,2 Gew.-% ES, 16,36 Gew.-% MAS und 0,69 Gew.-% MMA.

## Patentansprüche

1. Verfahren zur Aufkonzentrierung von Methacrylsäure in wasserhaltigen Methacrylsäurelösungen, dadurch gekennzeichnet, daß man die Lösung abkühlt, bis sich zwei Phasen ausbilden, und die an Methacrylsäure reichere Lösung von der an Methacrylsäure ärmeren Lösung abtrennt.

2. Verfahren nach Anspruch 1, dadurch gekennzeichnet, daß man auf 14 bis -2 °C abkühlt.

3. Verfahren nach Anspruch 1 oder 2, dadurch gekennzeichnet, daß man eine wasserhaltige Methacrylsäurelösung mit 13 bis 70 Gew.-% Methacrylsäure, bezogen auf 100 Gew.-% Methacrylsäure und Wasser, verwendet.

4. Verfahren nach einem der Ansprüche 1 bis 3, dadurch gekennzeichnet, daß man eine wasserhaltige Methacrylsäurelösung verwendet, die mittels eines thermischen Trennverfahrens vorkonzentriert worden ist.

5. Verfahren nach einem der vorhergehenden Ansprüche, dadurch gekennzeichnet, daß man nach der Phasentrennung die an Methacrylsäure ärmere Lösung auf eine Lösung mit 15 bis 20 Gew.-% Methacrylsäure, bezogen auf 100 Gew.-% Methacrylsäure und Wasser, mittels eines thermischen Trennverfahrens aufkonzentriert und die aufkonzentrierte Lösung wieder in die Abkühlung rückführt.

6. Verfahren nach einem der vorhergehenden Ansprüche, dadurch gekennzeichnet, daß man bei Vorhandensein von die Bildung von zwei Phasen verhindernden Nebenkomponenten einen oder mehrere mit Wasser eine Mischungslücke bildende Zusätze der Lösung zusetzt.

7. Verfahren nach Anspruch 6, dadurch gekennzeichnet, daß man der Lösung als Zusatz Ester der Methacrylsäure oder aliphatische Kohlenwasserstoffe zugibt.

8. Verfahren nach Anspruch 6 oder 7, dadurch gekennzeichnet, daß man der Lösung als Zusatz Methylmethacrylat zugibt.

9. Verfahren nach einem der vorhergehenden Ansprüche, dadurch gekennzeichnet, daß man eine wasserhaltige Methacrylsäurelösung verwendet, die zwischen 0 und 10 Gewichtsteile Verunreinigungen, bezogen auf 100 Gewichtsteile Methacrylsäure und Wasser, enthält.

10. Verfahren nach einem der vorhergehenden Ansprüche, dadurch gekennzeichnet, daß man eine wasserhaltige Methacrylsäurelösung verwendet, die Nebenkomponenten aus dem Herstellungsprozeß der Methacrylsäure enthält.
